# EUROPEAN PATENT APPLICATION

(11) **EP 4 516 116 A1**
(43) Date of publication of application: **05.03.2025**
(21) Application number: 23194035.4
(22) Date of filing: 29.08.2023
(51) Int. Cl.: A23L 2/46, A23L 3/04, A23L 3/22, A61L 2/04, C12H 1/18

(54) **TUNNEL PASTEURIZER WITH CENTRAL HEATING SYSTEM FOR A PLURALITY OF TUNNEL SECTIONS**

(71) Applicant: Sidel Participations, 76930 Octeville-sur-Mer (FR)
(72) Inventor: SOLFA, Andrea, 43126 Parma (IT)
(74) Representative: Sidel Group

(57) **Abstract**

It is described a tunnel Pasteurizer (1) for pasteurizing filled containers, comprising a heating system (4) for heating the fluids recirculating in all the recirculation circuits (314, 324, 334) of at least three sections of the tunnel (2) by withdrawing collected fluid from the specific collector (323) of a second section (22) of said sections, heating the withdrawn fluid, and inserting a respective portion of the withdrawn and heated fluid in each of said circuits (314; 324; 334) .

## Description

### TECHNICAL FIELD

The present invention relates to a pasteurizer for pasteurizing filled, which allows to have the pasteurizer working in a waiting mode more efficiently.

### BACKGROUND ART

It is known in the field of packaging pourable products by means of containers, to use pasteurizer for pasteurizing the filled containers. For example, the pasteurizer can be configured for pasteurizing closed bottles which are filled with beer or any other pourable product, or for pasteurizing sealed cans which are filled with beer or with any other pourable product.

It is known a type of pasteurizer which comprises a tunnel for conveying at least two opposite flows of containers to be pasteurized along the longitudinal extension of the tunnel. The two opposite flows comprise an upper flow and a lower flow. The tunnel comprises, along said extension, a first section, a second section and a third section which are distinct from each other. The three sections are configured for performing the pasteurization phase.

To this end, for each of said section, there is a respective fluid conditioning device which is associated to the respective section for thermally conditioning the containers flowing in the respective section.

Also, for each device, there is a respective first fluid dispenser for dispensing the conditioning fluid on the upper flow, a respective second fluid dispenser for dispensing the conditioning fluid on the lower flow, a respective collector for collecting the dispensed fluids having contacted the flows, and a respective recirculation circuit for recirculating the fluid between the collector and the dispensers.

There may be the need of stopping the flows of containers, and therefore of switching the pasteurizer from a production mode to a waiting mode. In this case, there is the further need of avoiding an overheating of the containers which are positioned in the sections mentioned above. To this end, the setpoint temperature of the fluid of each section is lowered. The second section is interposed between the first section and the third section. Therefore the containers of both the flows, which are located in the second section, have already a very high temperature, and therefore the cooling of the respective fluid is difficult. In each of the other sections, instead, at least the containers of one of the flows have a temperature which is still relatively low, so that the colling of the respective fluid is more easy.

### DISCLOSURE OF INVENTION

A pasteurizer according to present description or according to any of the appended pasteurizer claim is configured for allowing a better efficiency in the waiting mode of the pasteurizer.

The following brief description of the drawings and detailed description of the invention will be referred to a possible example embodiment of a pasteurizer according to present description.

### BRIEF DESCRIPTION OF THE DRAWINGS

The following detailed description will be referred to the accompanying drawings, in which:
Figure 1 is a schematic lateral view of the pasteurizer, along a longitudinal section.
Figure 2 shows a detail of Figure 1;
Figure 3 shows a graph with temperature trends.

### DETAILED DESCRIPTION OF THE INVENTION

The pasteurizer 1 is for pasteurizing a pourable product being packaged in containers. The pourable product is a pourable food product. To this end, the pasteurizer 1 is configured for thermal processing of containers containing the product, which are for example capped and filled bottles or sealed and filled cans. The containers are filled with a product which can be for example a food product. The food product can be for example beer.

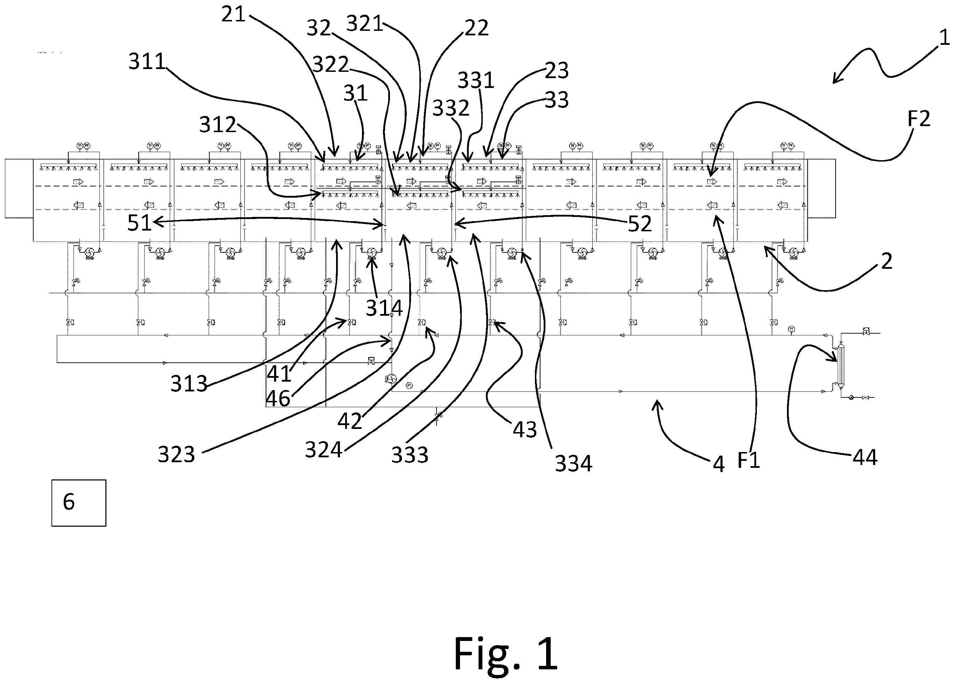

The pasteurizer comprises a tunnel 2 for conveying at least two opposite flows of containers to be pasteurized along the longitudinal extension of the tunnel 2. The two opposite flows comprise a lower flow F1 and an upper flow F2. In Figure 1, the lower flow F1 is from right to left, while the upper flow F2 is from left to right.

The tunnel 2 comprises, along said extension, a first section 21, a second section 22, and a third section 23 which are distinct from each other. Each section may comprise one or more subsections.

The second section 22 is longitudinally interposed between the first section 21 and the third section 23.

For each section 21 or 22 or 23, the pasteurizer 1 comprises a respective fluid conditioning device which is associated to the respective section for thermally conditioning the containers flowing in the respective section. The first conditioning device 31 is associated to the first section 21. The second conditioning device 32 is associated to the second section 22. The third conditioning device 33 is associated to the third section 23.

In each device 31 or 32 or 33, the pasteurizer 1 comprises a respective first fluid dispenser for dispensing the conditioning fluid on the upper flow F2. In each device 31 or 32 or 33, the pasteurizer 1 comprises a respective second fluid dispenser for dispensing the conditioning fluid on the lower flow F1. The first fluid dispenser of the first device 31 is indicated with 311. The second fluid dispenser of the first device 31 is indicated with 312. The first fluid dispenser of the second device 32 is indicated with 321. The second fluid dispenser of the second device 32 is indicated with 322. The first fluid dispenser of the third device 33 is indicated with 331. The second fluid dispenser of the third device 33 is indicated with 332.

In each device 31 or 32 or 33, the pasteurizer comprises a respective collector for collecting the dispensed fluids having contacted the flows of containers. The collector of the first device 31 is indicated with 313 and can be considered a first collector 313. The collector of the second device 32 is indicated with 323 and can be considered a second collector 323. The collector of the third device 33 is indicated with 333 and can be considered a third collector 333. In each device 31 or 32 or 33, the pasteurizer 1 comprises a respective recirculation circuit for recirculating the fluid between the collector and the dispensers of the same device. The recirculation circuit of the first device 31 is indicated with 314 and can be considered a first recirculation circuit 314. The recirculation circuit of the second device 32 is indicated with 324 and can be considered a second recirculation circuit 324. The recirculation circuit of the third device 33 is indicated with 334 and can be considered a third recirculation circuit 334.

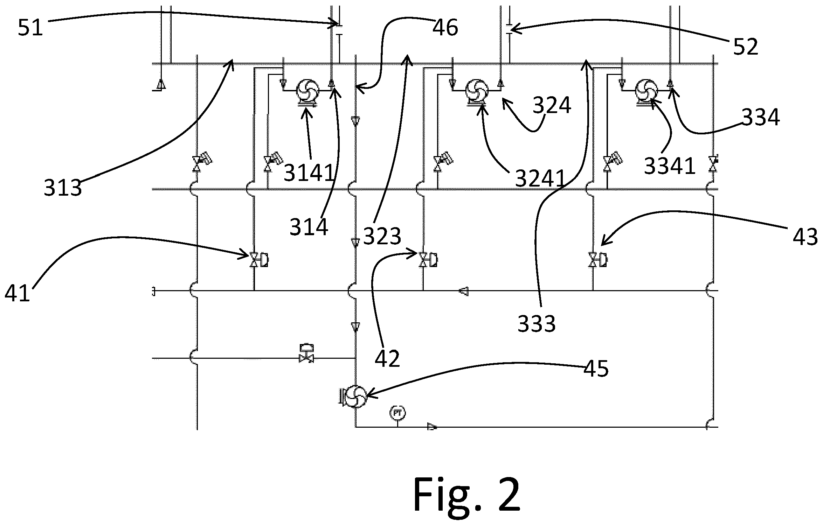

Each recirculation circuit comprises a respective pump for pumping the respective collected fluid. The pump of the first recirculation circuit 314 is indicated with 3141. The pump of the second recirculation circuit 324 is indicated with 3241. The pump of the third recirculation circuit 334 is indicated with 3341. The pumps of the recirculation circuits are indicated in Figure 2.

The pasteurizer 1 comprises a heating system 4 for heating the fluids recirculating in all said recirculation circuits 314, 324 and 334. To this end, the heating system withdraws collected fluid from the second collector 323, heats the withdrawn fluid, and inserts a respective portion of the withdrawn and heated fluid in each of the said recirculation circuits 314, 324 and 334.

Therefore the heating system 4 is used as a centralized heating system which can be employed to control actively the temperature of the fluids that are dispensed in all the sections, by heating the fluid collected in the second section 22. The mechanical complexity of the pasteurizer in this way is reduced.

The pasteurizer 1 comprises an automatic control device 6 which is configured for controlling automatically the heating system 4 as a function, for each section 21 or 22 or 23, of a respective setpoint temperature associated to the section 21 or 22 or 23. Each setpoint temperature associated to a section 21 or 22 or 23 corresponds to a desired temperature of the fluids which are dispensed in the section 21 or 22 or 23.

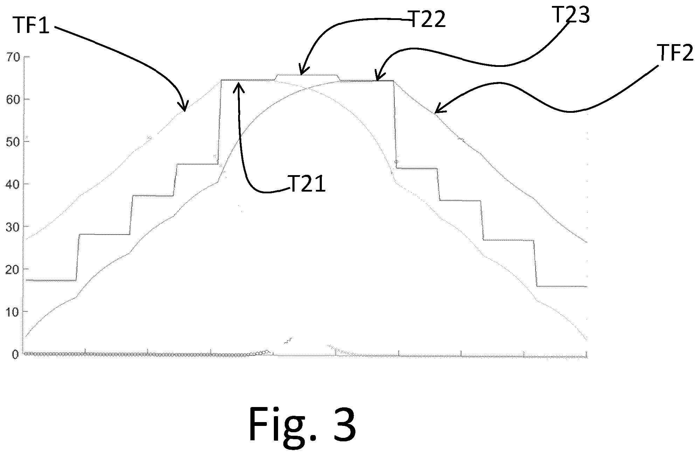

In Figure 3, the setpoint temperature associated to the first section is indicated with T21, and can be considered a first setpoint temperature T21. In Figure 3, the setpoint temperature associated to the second section is indicated with T22, and can be considered a second setpoint temperature T22. In Figure 3, the setpoint temperature associated to the third section is indicated with T23, and can be considered a third setpoint temperature T23.

The pasteurizer 1 is configured for operating selectively in a production mode or in a waiting mode.

The automatic control device 6 is configured for controlling automatically the speed of said flows so that the production mode corresponds to each flow having a bigger speed than in the waiting mode. In the waiting mode, for example, it can occur that the speed of the upper flow F2 and the speed of the lower flow F1 are zero or null.

The automatic control device 6 is configured so that each setpoint temperature is lowered in the waiting mode with respect to the production mode. For each section, the lowering of the setpoint temperature is performed by means of cooling the fluids dispensed in the same section. In this way, overheating of the containers can be avoided, to improve the quality of production.

Figure 3 can be considered to refer for example to the production mode.

The automatic control device 6 is preferably configured so that the second setpoint temperature T22, in the production mode, is higher than the first setpoint temperature T21 and higher than the third setpoint temperature T23.

For example, in the production mode the second setpoint temperature T22 may be about 64° Celsius, while the first setpoint temperature T21 and the third setpoint temperature may be for example 62° Celsius.

For example, in the waiting mode each of the first setpoint temperature T21, the second setpoint temperature T22, and the third setpoint temperature T23 may be 55° Celsius.

In Figure 3, TF1 indicates, for a container of the lower flow F1 in the production mode, a possible example trend of temperature as a function of the longitudinal position along the tunnel in the production mode. In Figure 3, TF2 indicates, for a container of the upper flow F2 in the production mode, a possible example trend of temperature as a function of the longitudinal position along the tunnel. In the production mode, the containers of both flows of containers have in the second section 22 a temperature which is relatively high, as shown for example in Figure 3. Therefore, in the waiting mode, the cooling of dispensed fluids in the second section 22 is more difficult than in the first section 21 and in the third section 23, because in each of first section 21 and third section 23, at least the containers of one of the flows still has a relatively low temperature in the production mode.

Moreover, the setpoint temperature in the production mode being higher in the second section 22 than in the first section 21 and in the third section 23, in the production mode, makes even more difficult reaching the lowered setpoint temperature of the waiting mode.

The heating system 4 comprises a heating device 44 for heating the fluid that the heating system 4 has withdrawn. The heating device 44 comprises a heat exchanger.

The heating system 4 is in fluid communication with the second collector 323 through a withdrawal line 46 for withdrawing the fluid to be heated. The withdrawal line 46 is distinct from the second recirculation circuit 324. The heating system 4 comprises the withdrawal line 46.

In this way, the flow rate of fluid withdrawn from the second collector 323 can be at least less related to or independent from the flow rate of fluid which enters in the second recirculation circuit 324.

The heating system 4 comprises a respective input valve for each recirculation circuit 314 or 324 or 334. The heating system 4 is in fluid communication with each recirculation circuit 314 or 324 or 334 by means of the respective input valve 41 or 42 or 43. Therefore, the heating system 4 is in fluid communication with the first recirculation circuit 314 by means of a first input valve 41. The heating system 4 is in fluid communication with the second recirculation circuit 324 by means of a second input valve 42. The heating system 4 is in fluid communication with the third recirculation circuit 334 by means of a third input valve 43.

The automatic control device 6 is configured for automatically controlling each input valve 41 or 42 or 43, as a function of the setpoint temperature associated to the respective section 21 or 22 or 23. In this way the automatic control device 6 can control the flow rate of each portion of heated fluid that is inserted in each recirculation circuit 314 or 324 or 334, to target the setpoint temperature of the respective section 21 or 22 or 23. The automatic control device 6 is configured for automatically controlling each input valve 41 or 42 or 43, as a function of the setpoint temperature associated to the respective section 21 or 22 or 23, preferably separately from the other input valves.

Each input valve 41 or 42 or 43 is a modulating valve. In this way the temperature in each section can be regulated more precisely and/or more finely.

The heating system 4 comprises a pump 45 for conveying the withdrawn fluid through the heat exchanger 44, to heat the withdrawn fluid by means of the heat exchanger 44. The pump 45 of the heating system 4 is indicated in Figure 2.

The automatic control device 6 is configured for automatically controlling the heat exchanger 44 and/or the pump 45 of the heating system 4, to control the temperatures of the dispensed fluids.

Each input valve 41 or 42 or 43 is configured for inputting the respective portion of heated fluid upstream the respective pump 3141 or 3241 or 3341. In this way, it is obtained a reduction of the impact on the action of the respective recirculation pump 3141 or 3241 or 3341, of the inputting of each portion of heated fluid in the respective recirculation circuit. In particular, inputting each portion upstream the respective pump allows to avoid any perturbation on the action of the pump which could be related to the high temperature of the inputted heated fluid.

The first collector 313 and the third collector 333 are in fluid communication with the second collector 323. Therefore, fraction of the fluid collected by the first collector 313 and a fraction of the fluid collected by the third collector 333 will be transferred, in particular in the waiting mode, to the second collector 323. In this way, the fluid collected by the first collector 313 and the fluid collected by the third collector 333, which are typically cooler, can help to cool the fluid which is dispensed in the second section 22, which would be, as said above, more difficult to cool, because the containers of both flows have a relatively high temperature in the second section 22. The transfer of said fractions of fluid from the first collector 313 and the third collector 333 to the second collector 323 can be produced at least or also because of the withdrawal of the fluid by the withdrawal line 46.

Also, this is helpful because in the waiting mode the setpoint temperature can be lowered later, in that the containers in the second section 22 need to complete the most relevant part of the pasteurization.

The first collector 313 can be in fluid communication with the second collector 323 by means of a first channel 51. The third collector 333 can be in fluid communication with the second collector 323 by means of a second channel 52. The pasteurizer 1 comprises the first channel 51 and the second channel 52.

For each section, the dispensed fluid is a liquid, for example water.

The first section 21, the second section 22 and the third section 23 may be considered as pasteurization sections.

The tunnel 2 may comprise other sections, for example preheating sections and/or cooling sections and/or other pasteurization sections.

Therefore, the pasteurizer is configured for performing more efficiently in the waiting mode, with a low mechanical complexity.

## Claims

1. - Pasteurizer (1) for pasteurizing a pourable product being packaged in containers, comprising:
- a tunnel (2) for conveying at least two opposite flows (F1, F2) of containers to be pasteurized along the longitudinal extension of the tunnel (2), the two opposite flows comprising an upper flow (F2) and a lower flow (F1) and the tunnel (2) comprising, along said extension, a first section (21), a second section (22) and a third section (23) which are distinct from each other;
- for each of said sections (21; 22; 23), a respective fluid conditioning device (31; 32; 33) which is associated to the respective section (21; 22; 23) for thermally conditioning the containers flowing in the respective section (21; 22; 23);
- in each device (31; 32; 33), a respective first fluid dispenser (311; 321; 331) for dispensing the conditioning fluid on the upper flow (F2), a respective second fluid dispenser (312; 322; 332) for dispensing the conditioning fluid on the lower flow (F1), a respective collector (313; 323; 333) for collecting the dispensed fluids having contacted the flows, and a respective recirculation circuit (314, 324; 334) for recirculating the fluid between the collector (313; 323; 333) and the dispensers (311-312; 321-322; 331-332) of the same conditioning device;
- a heating system (4) for heating the fluids recirculating at least in all said recirculation circuits (314, 324, 334) by withdrawing collected fluid from the specific collector (323) of the device (32) associated to the second section (22), heating the withdrawn fluid, and inserting a respective portion of the withdrawn and heated fluid in each of said circuits (314; 324; 334).

2. Pasteurizer (1) according to claim 1, wherein the heating system (4) comprises a heating device (44) for heating the withdrawn fluid, the heating device (44) comprising preferably a heat exchanger.

3. Pasteurizer (1) according to claim 2, wherein the heating system (4) is in fluid communication with said specific collector (323) through a withdrawal line (46) for withdrawing the fluid to be heated, the withdrawal line (46) being distinct from the recirculation circuit (324) associated to the second section (22), the heating system (4) comprising the withdrawal line (46).

4. Pasteurizer (1) according to Claim 3, wherein the heating system (4) comprises a respective input valve (41; 42; 43) for each recirculation circuit (314; 324; 334), the heating system (4) being in fluid communication with each recirculation circuit (314; 324; 334) by means of the respective input valve (41; 42; 43).

5. Pasteurizer (1) according to Claim 4, wherein each input valve (41; 42; 43) is a modulating valve.

6. Pasteurizer (1) according to Claim 4 or 5, wherein each recirculation circuit (314; 324; 334) comprises a respective pump (3141; 3241; 3341) for pumping the respective collected fluid, each input valve (41; 42; 43) being configured for inputting the respective portion upstream the respective pump (3141; 3241; 3341).

7. Pasteurizer (1) according to any of the previous claims, wherein the heating system (4) comprises a pump (45) for conveying the withdrawn fluid through the heating device (44), to heat the fluid by means of the heating device (44).

8. Pasteurizer (1) according to any of the previous claims, wherein the collector (313) associated to the first section (21) and the collector (333) associated to the third section (23) are in fluid communication with the collector (323) associated to the second section (22).

9. Pasteurizer (1) according to any of the previous claims, comprising an automatic control device (6) which is configured for controlling automatically the heating system (4) as a function, for each section (21; 22; 23), of a respective setpoint temperature (T21; T22; T23) associated to the section (21; 22; 23), each setpoint temperature corresponding to the desired temperature of the fluids being dispensed in the respective section (21; 22; 23);
wherein the pasteurizer (1) is configured for operating selectively in a production mode or in a waiting mode;
wherein the automatic control device (6) is configured for controlling automatically the speed of said flows so that the production mode corresponds to each flow (F1; F2) having a bigger speed in the production mode than in the waiting mode;
wherein the automatic control device (6) is configured so that each setpoint temperature is lowered in the waiting mode with respect to the production mode.

10. Pasteurizer (1) according to Claims 4 or and Claim 9, wherein the automatic control device (6) is configured for automatically controlling each input valve (41; 42; 43), as a function of the setpoint temperature associated to the respective section (21; 22; 23) and preferably separately from the other input valves.

11. Pasteurizer (1) according to Claim 9 or 10, wherein the automatic control device (6) is configured so that, in the production mode, the setpoint temperature (T22) of the second section (22) is higher than the setpoint temperature (T21) of the first section (21) and higher than the setpoint temperature (T23) of the third section (23).

12. Pasteurizer (1) according to any of the previous Claims, wherein the second section (22) is longitudinally interposed between the first section (21) and the third section (23).

13. Pasteurizer (1) according to any of the previous claims, wherein said fluid is a liquid.

14. Pasteurizer (1) according to any of the previous Claims, wherein the pasteurizer is configured for pasteurizing filled and closed containers, for example capped and filled bottles or sealed and filled cans.

15. Pasteurizer (1) according to any of the previous Claims, wherein each section (21; 22; 23) comprises one or more respective subsections.
